# DEMANDE DE BREVET EUROPEEN

(11) **EP 2 324 791 A1**
(43) Date de publication de la demande: **25.05.2011**
(21) Numéro de dépôt: 10168681.4
(22) Date de dépôt: 07.07.2010
(51) Int. Cl.: A61B 19/00, G06K 19/077

(54) **Procédé d'intégration d'un composant RFID dans un substitut de prothèse ou d'implant ou dans un instrument chirurgical en polymère ou en plastique**

(30) Priorité: 08.07.2009 FR 0903372
(71) Demandeur: SFERIC STELLITE, 41500 Menars (FR)
(72) Inventeur: Grimard, Jean-Christophe, 41120 Cellettes (FR)
(74) Mandataire: Debay, Yves

(57) **Abrégé**

L'invention consiste en un procédé d'intégration d'un composant RFID (3) dans un substitut (01, 02) d'implant ou de prothèse ou dans un instrument chirurgical comprenant au moins une étape d'usinage ou d'alésage du corps du substitut (01, 02) ou de l'instrument chirurgical pour former un logement (1) puis une cavité (2) destiné à recevoir un couvercle (4) du logement (1) ; caractérisé en ce qu'il comprend en outre une étape de soudage par ultrasons à l'aide d'une sonotrode du couvercle (4) au corps du substitut (01) ou de l'instrument chirurgical en polymère ou en plastique de façon à ce que la surface externe du substitut (01, 02) ou de l'instrument chirurgical ne présente aucune aspérité saillante et que la soudure (61, 62) obtenue soit étanche et durable. L'invention concerne également le substitut (01, 02) ou l'instrument chirurgical.

## Description

La présente invention concerne le domaine des substituts de prothèse ou d'implant ou des instruments chirurgicaux. La présente invention propose plus particulièrement un procédé d'intégration d'un composant RFID dans un substitut de prothèse ou d'implant ou dans un instrument chirurgical en polymère ou en plastique ou dans la portion en polymère ou en plastique d'un substitut de prothèse ou d'implant ou d'un instrument chirurgical.

Les instruments médicaux, et plus particulièrement, les substituts de prothèse ou d'implant et les instruments chirurgicaux, font généralement l'objet d'un suivi rigoureux destiné à garantir leurs conditions d'utilisation de décontamination et la sécurité des patients. Ce suivi consiste à enregistrer et mettre à jour toute information utile portant sur l'instrument, par exemple, son numéro de série, l'historique clinique des patients opérés, les spécifications d'utilisation et de maintenance, les dates de ses opérations de maintenance ou de stérilisation, le nombre d'utilisations, etc. À cet effet, les composants RFID (en anglais, radio frequency identification) qui peuvent englober les transpondeurs ou tags ou les étiquettes RFID sont largement utilisées car elles présentent des avantages considérables. Solidaires d'un instrument, elles permettent, en association avec un module de lecture et/ou écriture doté d'une antenne et un ordinateur central, l'écriture, la lecture et le stockage d'un grand nombre de données relatives à cet instrument. L'enregistrement d'une information peut être réalisé automatiquement, lors d'une étape de nettoyage, par exemple, de façon à limiter l'erreur d'origine humaine. Un tel mode de fonctionnement est décrit, par exemple, dans le document EP 0 992 212 pour un instrument métallique. Les étiquettes électroniques sont, en outre, extrêmement fiables et supportent les températures élevées des procédés de stérilisation.

Dans l'art antérieur, l'intégration de composants RFID dans des instruments chirurgicaux métalliques est donc bien connue. Or, l'instrumentation chirurgicale utilise aussi des instruments en polymère ou en plastique qui constituent 20% environ de l'instrumentation chirurgicale. En parallèle, des substituts d'implants ou de prothèses sont des organes le plus souvent en plastique ou en polymère qui sont souvent utilisés comme composant d'essai pour, par exemple, assurer l'intégration optimale d'un implant ou prothèse. Le chirurgien doit récréer l'environnement de la prothèse définitive dans son milieu de destination pour qu'elle soit bien stabilisée et assure correctement sa fonction biomécanique : les axes, les dimensions de la prothèse doivent tenir compte, entre autres, de la tension des ligaments et des muscles pour une bonne stabilité de la prothèse. Il utilise alors des composants d'essai de différentes caractéristiques le plus souvent en polymère ou en plastique qui simulent les composants définitifs et leur assemblage.

Comme pour les instruments métalliques, il est nécessaire d'identifier les instruments ou substituts en polymère ou en plastique de la même manière de façon à identifier, par exemple, la taille et les propriétés dimensionnelles du composant d'essai qui détermineront celles de la prothèse. Cependant, de par sa matière, l'instrument chirurgical ou le substitut en polymère ou en plastique ne permet pas l'intégration de composants RFID avec les mêmes techniques que les instruments métalliques, principalement pour permettre le maintien de l'étanchéité et la tenue malgré des stérilisations successives.

En effet, les propriétés physiques des polymères ou des plastiques imposent des contraintes qui n'existent pas pour des métaux.

Par exemple, les polymères ou les plastiques subissent des dilations plus importantes que des métaux sous l'effet de la chaleur et ils sont plus sensibles à l'humidité ; ce qui pose problème lors des stérilisations des instruments. Par conséquent, le collage d'un couvercle sur un logement contenant un composant RFID qui est une solution pour les instruments métalliques n'est pas une solution efficace. De par la dilation du polymère ou du plastique et l'incidence de l'humidité, la colle endure des contraintes qui la rendent inefficace et donc nuit à l'étanchéité du logement contenant le composant RFID qui n'est plus fonctionnel au bout d'une dizaine d'utilisations.

Le soudage par laser ou par faisceau d'électrons est une autre solution utilisée pour les instruments métalliques pour fixer le couvercle sur le logement. Or, le plastique a des propriétés optiques qui empêchent une soudure efficace. La plus grande partie du faisceau laser ou à électrons est réfléchie. La partie restante du faisceau n'est pas assez efficace pour souder le couvercle sur le logement.

On ne peut donc pas répéter les techniques utilisées sur les métaux pour les polymères ou les plastiques.

La présente invention a donc pour objet de palier un ou plusieurs des inconvénients de l'art antérieur en définissant un procédé d'intégration d'un composant RFID dans substitut d'implant ou de prothèse ou dans un instrument chirurgical en polymère ou en plastique.

À cet effet, l'invention concerne un procédé d'intégration d'un composant RFID dans un substitut d'implant ou de prothèse ou dans un instrument chirurgical qui comprend au moins :
- une étape d'usinage ou d'alésage du corps du substitut ou de l'instrument chirurgical pour former un logement destiné à recevoir un composant RFID ;
- une étape d'usinage ou d'alésage du corps du substitut ou de l'instrument chirurgical pour former une cavité destinée à recevoir un couvercle du logement ;
- une étape de mise en place du composant RFID dans le logement ;
- une étape de mise en place du couvercle dans la cavité ; caractérisé en ce qu'il comprend en outre :
- une étape de soudage par ultrasons à l'aide d'une sonotrode du couvercle en polymère ou en plastique au corps du substitut d'implant ou de prothèse en polymère ou en plastique ou de l'instrument chirurgical en polymère ou en plastique de façon à ce que la surface externe du substitut ou de l'instrument chirurgical ne présente aucune aspérité saillante et que la soudure obtenue soit étanche et durable.

Selon une autre particularité, le couvercle a un diamètre inférieur au diamètre de la cavité.

Selon une autre particularité, le couvercle a un diamètre supérieur ou égal au diamètre la cavité.

Selon une autre particularité, la soudure est réalisée entre la partie inférieure du couvercle et le fond de la cavité.

Selon une autre particularité, la soudure est réalisée entre la partie inférieure du couvercle et le fond de la cavité et entre le pourtour du couvercle et la paroi verticale de la cavité.

Selon une autre particularité, lors de l'étape de soudage, la sonotrode est placée à cheval entre le couvercle et le corps du substitut ou de l'instrument chirurgical.

Selon une autre particularité, la tête de la sonotrode a une surface de contact avec les parties à souder du substitut ou de l'instrument chirurgical lisse ou moletée.

Selon une autre particularité, le couvercle est plus épais en son centre qu'en sa périphérie, l'épaisseur du couvercle diminuant progressivement au fur et à mesure que l'on s'approche de sa périphérie.

Selon une autre particularité, le couvercle et le corps du substitut ou de l'instrument chirurgical sont de la même matière ou de matières ayant des propriétés viscoélastiques et thermiques semblables.

Un autre but de l'invention est atteint en proposant un substitut d'implant ou de prothèse ou un instrument chirurgical comprenant un logement formé par usinage destiné à recevoir à l'intérieur un composant RFID et une cavité destinée à la fermeture du logement de façon étanche et durable par un couvercle caractérisé en ce que le substitut d'implant ou de prothèse ou l'instrument chirurgical est en polymère ou en plastique et en ce que le couvercle est soudé par ultrasons.

Selon une autre particularité, la soudure est réalisée entre la partie inférieure du couvercle et le fond de la cavité.

Selon une autre particularité, la soudure est réalisée entre la partie inférieure du couvercle et le fond de la cavité et entre le pourtour du couvercle et la paroi verticale de la cavité.

L'invention sera mieux comprise et d'autres buts, caractéristiques, détails et avantages de celle-ci apparaîtront plus clairement au cours de la description explicative qui va suivre faite en référence aux figures annexées données à titre d'exemples non limitatifs dans lesquelles :
- la figure 1a représente un substitut d'implant ou de prothèse en polymère ou en plastique qui est une embase tibiale d'essai avec un composant RFID intégré selon le procédé de l'invention.
- la figure 1b représente un substitut d'implant ou de prothèse en polymère ou en plastique qui est une tête fémorale d'essai avec un composant RFID intégré selon le procédé de l'invention.
- la figure 1c représente une partie en polymère ou en plastique d'un instrument chirurgical avec un composant RFID intégré selon le procédé de l'invention.
- la figure 2a représente une vue du dessus de l'embase tibiale d'essai avec l'emplacement de la cavité et du logement selon une configuration.
- la figure 2b représente une vue de face et en coupe de l'embase tibiale d'essai avec la cavité et le logement contenant le composant RFID selon une configuration.

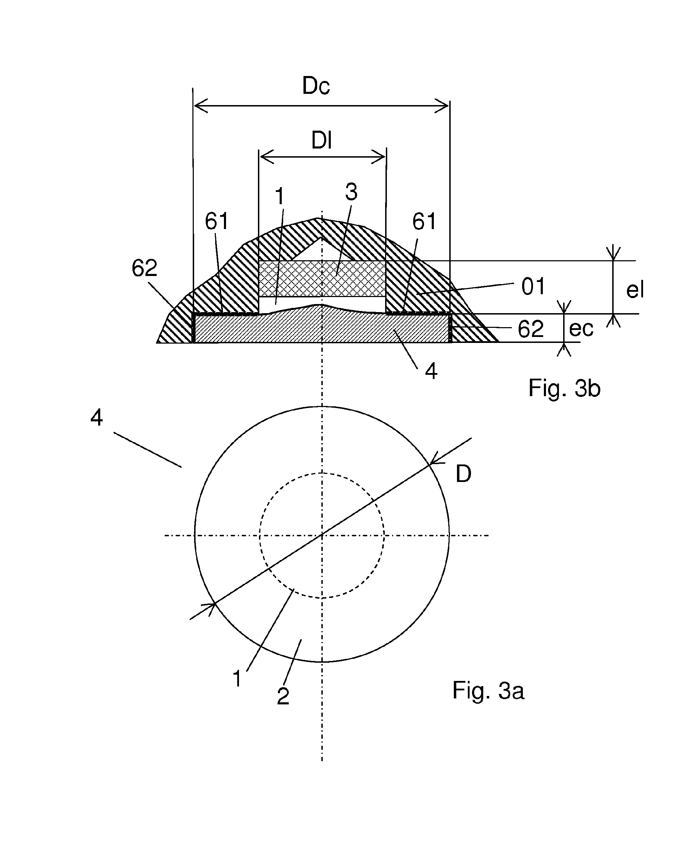

- la figure 3a représente une vue du dessus du couvercle.
- la figure 3b représente une vue en coupe du logement et de la cavité contenant le composant RFID et fermé par un couvercle.
- la figure 4 représente un organigramme illustrant le procédé selon l'invention.

Dans la suite, le terme « composant RFID » désignera tout type de composant RFID tels que le transpondeur ou l'étiquette RFID.

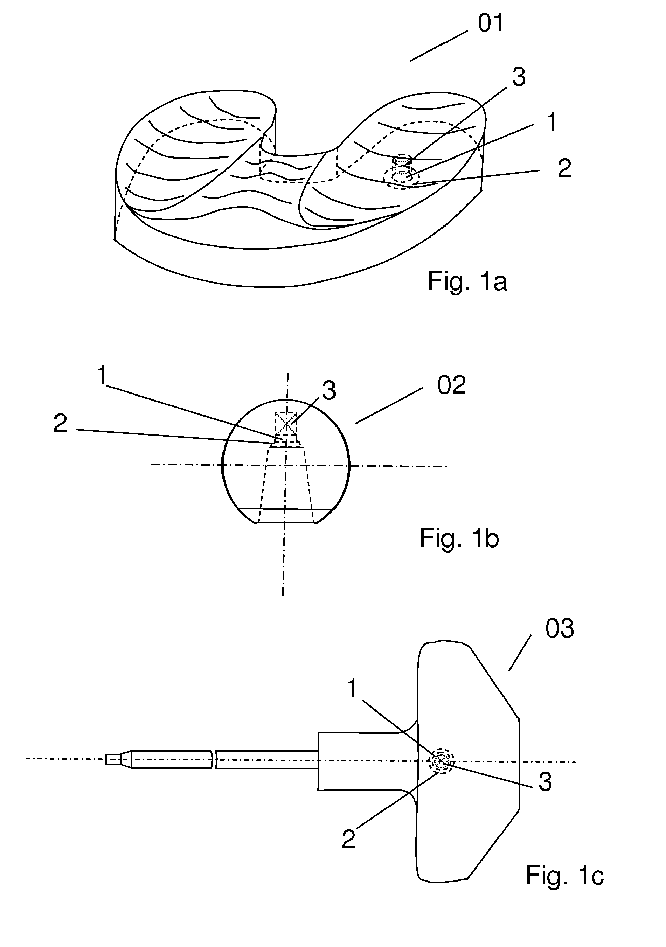

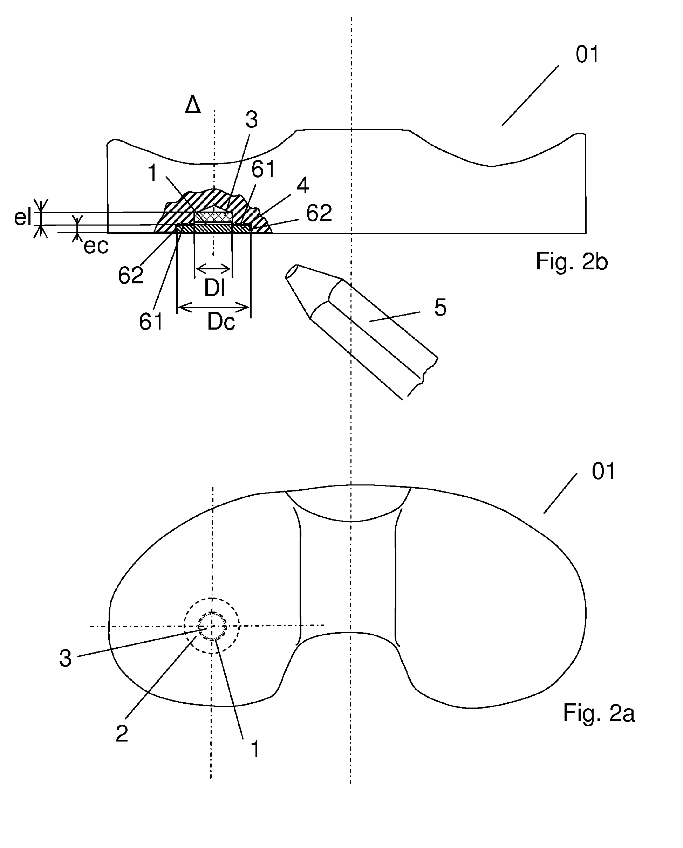

En référence aux figures, l'invention proposée permet d'intégrer un composant RFID (3) dans un substitut (01, 02) d'implant ou de prothèse ou dans un instrument chirurgical (03) en polymère ou en plastique. Dans la suite de la description, les termes « instrument chirurgical » et « substitut d'implant ou de prothèse » doivent être pris de manière non-limitative. En effet, ces termes peuvent englober un substitut d'implant ou de prothèse ou un instrument chirurgical entièrement en polymère ou en plastique ou alors un substitut d'implant ou de prothèse ou un instrument chirurgical en partie en polymère ou en plastique. À titre d'exemples représentés aux figures 1a et 1b, le substitut d'implant ou de prothèse peut être une embase tibiale (01) d'essai (figure 1a) ou une tête fémorale (02) d'essai (figure 1b). À la figure 1c, l'instrument chirurgical peut être une poignée (03) de manipulation en polymère ou en plastique d'un instrument chirurgical complet. Dans la suite des figures 2a et 2b, n'est représenté que l'embase tibiale (01) par souci de clarté et non de limitation.

De manière non-limitative, les polymères constituant le substitut (01, 02) d'implant ou de prothèse ou l'instrument chirurgical (03) peuvent être le polyacétal (POM), le polysulfone (PSU), le polyphénylsulfone (PPSU), le polyéthersulfone (PES), le polyphénylèneéther (PPE), le polyétherimide (PEI), le polyétherétherkétone (PEEK) ou le polyétherkétonekétone (PEKK).

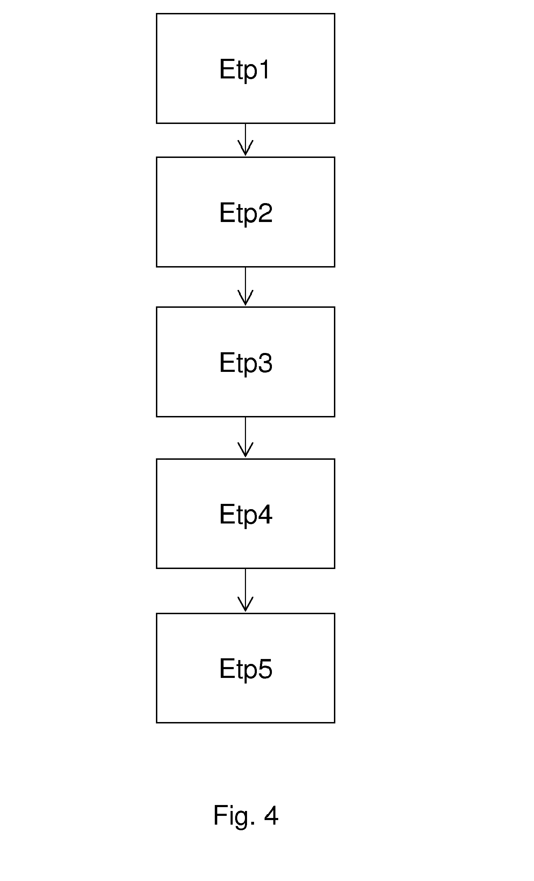

En référence à la figure 4, l'invention concerne un procédé d'intégration d'un composant RFID (3) dans un substitut (01, 02) d'implant ou de prothèse ou dans un instrument chirurgical (03) en polymère ou en plastique qui comprend au moins les étapes suivantes :
Une étape (Etp1) d'usinage ou d'alésage du corps du substitut (01, 02) d'implant ou de prothèse ou de l'instrument chirurgical (03) pour former un logement (1) de forme sensiblement cylindrique destiné à recevoir un composant RFID (3).

Dans une étape (Etp2) suivante, un usinage ou un alésage est également réalisé pour former une cavité (2) sensiblement cylindrique destinée à recevoir le couvercle (4) fermant le logement (1).

Les usinages et les alésages pour former le logement (1) et la cavité (2) sont réalisés selon un axe (Δ) perpendiculaire à la surface externe du substitut (01, 02) d'implant ou de prothèse ou de l'instrument chirurgical (03).

Il est à noter que le diamètre (DI) de l'alésage formant le logement (1) est inférieur au diamètre (Dc) de l'alésage formant la cavité (2) pour le couvercle (4). En outre, l'épaisseur ou la profondeur (el) de l'alésage formant le logement (1) est supérieure à la profondeur (ec) de l'alésage formant la cavité (2) pour le couvercle (4).

En fonction de la zone du corps du substitut (01, 02) d'implant ou de prothèse ou de l'instrument chirurgical (03) qui est usinée et des dimensions du composant RFID (3), l'alésage pour former le logement (1) doit être réalisé selon une profondeur (el) sensiblement à proximité de la surface externe du corps du substitut (01, 02) d'implant ou de prothèse ou de l'instrument chirurgical (03).

L'épaisseur du couvercle (4) et les dimensions du logement (1) permettent de positionner le composant RFID (3) sensiblement à proximité de la surface externe du substitut (01, 02) d'implant ou de prothèse ou de l'instrument chirurgical (03) de façon à ce que les informations contenues dans le composant RFID (3) puissent être lues par un système de lecture (5). L'épaisseur du couvercle (4) doit aussi autoriser le système de lecture (5) à envoyer des informations à stocker dans le composant RFID (3). Le couvercle (4) a une épaisseur qui peut varier de 0,7 mm à 1,3 mm. Avantageusement, le couvercle (4) a une épaisseur de 1 mm. Le couvercle (4) a un diamètre (D) qui peut varier de 9,97 mm à 10,02 mm. Avantageusement, le couvercle (4) a un diamètre (D) de 10 mm.

Le couvercle (4) et le corps du substitut (01, 02) d'implant ou de prothèse ou de l'instrument chirurgical (03) sont de matières ayant des propriétés viscoélastiques et thermiques semblables.

De façon avantageuse, le couvercle (4) et le substitut d'implant ou de prothèse (01, 02) ou le corps de l'instrument chirurgical (03) sont de la même matière.

Dans une étape (Etp3) suivante, le composant RFID (3) est mis en place dans le logement (1).

Dans une étape (Etp4) suivante, le couvercle (4) est mis en place dans la cavité (2).

Dans une configuration, le couvercle (4) a un diamètre (D) plus petit que le diamètre (Dc) de la cavité (2). Le jeu résultant entre les bords du couvercle (4) et les parois latérales de la cavité (2) ne dépasse pas 1/10 de millimètre. Avantageusement, le jeu entre les bords du couvercle (4) et les parois latérales de la cavité (2) ne dépasse pas 1/20 de millimètre.

Dans une autre configuration préférée, le couvercle (4) a un diamètre (D) égal ou supérieur au diamètre (Dc) de la cavité (2). Le diamètre (D) du couvercle (4) ne dépasse pas le diamètre (Dc) de la cavité (2) de plus de 1/20 de millimètre. Avantageusement, le diamètre (D) du couvercle (4) ne dépasse pas le diamètre (Dc) de la cavité (2) de plus de 1/40 de millimètre. De préférence, le diamètre (D) du couvercle (4) est égal au diamètre (Dc) de la cavité (2). Dans cette configuration, l'élasticité du polymère ou du plastique permet l'insertion du couvercle (4) dans la cavité (2).

Dans une configuration et de manière non-limitative, la cavité (2) a un diamètre (Dc) compris entre 9,98 mm et 10,03 mm et avantageusement, le diamètre (Dc) de la cavité (2) est égal à 10 mm. Dans cette même configuration, le logement (1) a un diamètre (DI) de 5,6 mm et une épaisseur ou profondeur (el) de 2 mm avec un composant RFID (3) de diamètre 5,6 et une épaisseur de 1,7 mm.

Dans une étape (Etp5) suivante, le couvercle (4) est soudé au corps du substitut (01, 02) d'implant ou de prothèse ou de l'instrument chirurgical (03) par une étape (Etp5) de soudage par ultrasons.

Le soudage par ultrasons est une technique industrielle où des vibrations ultrasonores à hautes fréquences sont appliquées localement sur deux parties à souder pour créer une soudure à l'état solide via un champ de pression et un échauffement localisé. Selon le réglage de la sonotrode, les fréquences d'utilisation peuvent être comprises dans un intervalle de 2 kHz à 40 kHz avec des amplitudes adaptées en fonction du type de polymère ou de plastique constituant le couvercle (4) et le substitut d'implant ou de prothèse (01, 02) ou l'instrument chirurgical (03).

Il est à noter que la sonotrode utilisée pour le soudage peut avoir une tête, partie qui entre en contact avec les pièces à souder, lisse, striée ou moletée. La sonotrode à tête moletée ou striée ajoute à l'efficacité des ultrasons par un écrouissage mécanique. Ce relief de surface de la tête de la sonotrode permet d'améliorer l'interpénétration du ou des matériaux du couvercle (4) et du substitut d'implant ou de prothèse (01, 02) ou l'instrument chirurgical (03).

Les paramètres contrôlant la soudure sont la fréquence des ultrasons, la pression de la tête de la sonotrode sur les parties à souder, le temps d'application de la sonotrode et la forme de la sonotrode.

Par exemple de façon non limitative, pour un corps du substitut (01, 02) d'implant ou de prothèse ou de l'instrument chirurgical (03) et un couvercle (4) en polyacétal, l'énergie à appliquer est de 100 J de fréquence de 300 Hz avec une force de pression de 200 N pendant 2,8 s.

La surface de contact de la sonotrode avec la zone à souder est, de préférence, annulaire.

De façon avantageuse, la tête de la sonotrode est placée à cheval entre le couvercle (4) et le corps du substitut (01, 02) d'implant ou de prothèse ou de l'instrument chirurgical (03) lors de l'étape de soudage en venant appuyer sur le couvercle (4). Au moment du soudage, la tête de la sonotrode doit donc avoir une surface de contact couvrant à la fois la zone périphérique du couvercle (4) et la zone autour de la cavité (2) destinée à recevoir le couvercle (4) du corps du substitut (01, 02) d'implant ou de prothèse ou de l'instrument chirurgical (03). La tête de la sonotrode n'est pas appliquée sur toute la surface du couvercle (4). Cette configuration de la tête de sonotrode permet d'éliminer toute aspérité saillante ou un épaulement entre le couvercle (4) et le corps du substitut (01, 02) d'implant ou de prothèse ou de l'instrument chirurgical (03).

Dans la configuration où le couvercle (4) a un diamètre (D) plus petit que le diamètre (Dc) de la cavité (2), la soudure est créée aux surfaces (61) de contact du couvercle (4) et de la cavité (2), c'est-à-dire sur la partie inférieure du couvercle (4) et le fond de la cavité (2).

Dans la configuration où le couvercle (4) a un diamètre (D) égal ou supérieur au diamètre (Dc) de la cavité (2), la soudure est créée aux surfaces (61 et 62) de contact du couvercle (4) et de la cavité (2), c'est-à-dire entre la partie inférieure du couvercle (4) et le fond de la cavité (2) ainsi que entre le pourtour du couvercle (4) et pourtour ou la paroi verticale de la cavité (2). Cette dernière configuration permet une étanchéité optimale du logement (1). Préférentiellement, il doit donc y avoir le plus large contact possible entre le couvercle (4) et la cavité (2). La différence entre le diamètre (Dc) de la cavité et le diamètre (DI) du logement est supérieure ou égale à 3 mm pour avoir une surface de contact optimale entre la partie inférieure du couvercle (4) et le fond de la cavité (2) et ainsi améliorer l'étanchéité du logement (1).

Les soudures (61, 62) aux zones de contact du couvercle (4) et de la cavité (2) du substitut (01, 02) d'implant ou de prothèse de l'instrument chirurgical (03) sont donc réalisées par échauffement local, affaissement et éventuellement écrouissage des matières.

De façon avantageuse, le couvercle (4) est renforcé en son centre. Pour cela, l'épaisseur du couvercle (4) est plus importante en son centre qu'en sa périphérie. L'épaisseur du couvercle diminuant progressivement au fur et à mesure que l'on s'approche de sa périphérie. De façon non limitative, le couvercle (4) a une épaisseur augmentée de 0,5 mm au centre par rapport à son épaisseur en périphérie. Ceci permet une soudure du couvercle (4) dans la cavité (2) plus efficace et plus étanche.

Ce procédé permet d'obtenir un substitut (01, 02) d'implant ou de prothèse en polymère ou en plastique ou instrument chirurgical (03) en polymère ou en plastique comprenant un logement (1) formé par usinage destiné à recevoir à l'intérieur un composant RFID (3) et une cavité (2) destinée à la fermeture du logement (1) de façon étanche et durable par un couvercle (4) en polymère ou en plastique soudé par ultrasons au corps du substitut (01, 02) d'implant ou de prothèse ou de l'instrument chirurgical (03).

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Procédé d'intégration d'un composant RFID (3) dans un substitut (01, 02) d'implant ou de prothèse ou dans un instrument chirurgical (03) comprenant au moins :
- une étape (Etp1) d'usinage ou d'alésage du corps du substitut (01, 02) d'implant ou de prothèse ou de l'instrument chirurgical (03) pour former un logement (1) destiné à recevoir un composant RFID (3) ;
- une étape (Etp2) d'usinage ou d'alésage du corps du substitut (01, 02) d'implant ou de prothèse ou de l'instrument chirurgical (03) pour former une cavité (2) destinée à recevoir un couvercle (4) du logement (1) ;
- une étape (Etp3) de mise en place du composant RFID (3) dans le logement (1) ;
- une étape (Etp4) de mise en place du couvercle (4) dans la cavité (2);
**caractérisé en ce qu'**il comprend en outre :
- une étape (Etp5) de soudage par ultrasons à l'aide d'une sonotrode du couvercle (4) en polymère ou en plastique au corps du substitut (01, 02) d'implant ou de prothèse en polymère ou en plastique ou de l'instrument chirurgical (03) de façon à ce que la surface externe du substitut (01, 02) d'implant ou de prothèse ou de l'instrument chirurgical (03) ne présente aucune aspérité saillante ou un épaulement entre le couvercle (4) et le corps du substitut (01, 02) d'implant ou de prothèse ou de l'instrument chirurgical (03) et que la soudure (61, 62) obtenue soit étanche et durable.

2. Procédé selon la revendication 1, **caractérisé en ce que** le couvercle (4) a un diamètre inférieur au diamètre de la cavité (2).

3. Procédé selon la revendication 1, **caractérisé en ce que** le couvercle (4) a un diamètre supérieur ou égal au diamètre de la cavité (2).

4. Procédé selon les revendications 1 et 2, **caractérisé en ce que** la soudure (61) est réalisée entre la partie inférieure du couvercle (4) et le fond de la cavité (2).

5. Procédé selon les revendications 1 et 3, **caractérisé en ce que** la soudure (61, 62) est réalisée (61) entre la partie inférieure du couvercle (4) et le fond de la cavité (2) et (62) entre le pourtour du couvercle (4) et la paroi verticale de la cavité (2).

6. Procédé selon les revendications 1 à 5, **caractérisé en ce que**, lors de l'étape (Etp5) de soudage, la sonotrode est placée à cheval entre le couvercle (4) et le corps du substitut (01, 02) d'implant ou de prothèse ou de l'instrument chirurgical (03) telle façon que la tête de la sonotrode a une surface de contact couvrant à la fois la zone périphérique du couvercle (4) et la zone autour de la cavité (2) destinée à recevoir le couvercle (4) du corps du substitut (01, 02) d'implant ou de prothèse ou de l'instrument chirurgical (03)..

7. Procédé selon les revendications 1 à 6, **caractérisé en ce que** la tête de la sonotrode a une surface de contact avec les parties du substitut (01, 02) d'implant ou de prothèse ou de l'instrument chirurgical (03) à souder lisse ou moletée.

8. Procédé selon les revendications 1 à 7, **caractérisé en ce que** le couvercle (4) est plus épais en son centre qu'en sa périphérie, l'épaisseur du couvercle (4) diminuant progressivement au fur et à mesure que l'on s'approche de sa périphérie.

9. Procédé selon les revendications 1 à 8, **caractérisé en ce que** le couvercle (4) et le corps du substitut (01, 02) d'implant ou de prothèse ou de l'instrument chirurgical (03) sont de la même matière ou de matières ayant des propriétés viscoélastiques semblables.

10. Substitut (01, 02) d'implant ou de prothèse ou instrument chirurgical (03) comprenant un logement (1) formé par usinage destiné à recevoir à l'intérieur un composant RFID (3) et une cavité (2) destinée à la fermeture du logement (1) de façon étanche par un couvercle (4) **caractérisé en ce que** le substitut (01, 02) d'implant ou de prothèse ou l'instrument chirurgical (03) est en polymère ou en plastique et **en ce que** le couvercle (4) est soudé par ultrasons au corps du substitut (01, 02) d'implant ou de prothèse ou de l'instrument chirurgical (03).

11. Substitut (01, 02) d'implant ou de prothèse ou instrument chirurgical (03) selon la revendication 10 **caractérisé en ce que** la soudure (61) est réalisée entre la partie inférieure du couvercle (4) et le fond de la cavité (2).

12. Substitut (01, 02) d'implant ou de prothèse ou instrument chirurgical (03) selon la revendication 10 **caractérisé en ce que** la soudure (61, 62) est réalisée entre la partie inférieure du couvercle (4) et le fond de la cavité (2) et entre le pourtour du couvercle (4) et la paroi verticale de la cavité (4).
